# EUROPÄISCHE PATENTSCHRIFT

(11) **EP 3 848 305 B1**
(45) Veröffentlichungstag und Bekanntmachung des Hinweises auf die Patenterteilung: **16.04.2025**
(21) Anmeldenummer: 20151151.6
(22) Anmeldetag: 10.01.2020
(51) Int. Cl.: B65G 1/04, B65G 1/137, G16H 20/13

(54) **VERFAHREN ZUM BETREIBEN EINER KOMMISSIONIERVORRICHTUNG FÜR ARZNEIMITTEL SOWIE KOMMISSIONIERVORRICHTUNG ZUM DURCHFÜHREN DES VERFAHRENS**
METHOD FOR OPERATING A PICKING DEVICE FOR MEDICAMENTS AND PICKING DEVICE FOR CARRYING OUT THE METHOD
PROCÉDÉ DE FONCTIONNEMENT D'UN DISPOSITIF DE PRÉPARATION DE COMMANDES POUR MÉDICAMENTS AINSI QUE DISPOSITIF DE PRÉPARATION DE COMMANDES PERMETTANT DE METTRE EN OEUVRE LE PROCÉDÉ

(43) Veröffentlichungstag der Anmeldung: 14.07.2021
(73) Patentinhaber: Becton Dickinson Rowa Germany GmbH, 53539 Kelberg (DE)
(72) Erfinder: HOLZHÄUSER, Dennis, 56743 Mendig (DE)
(74) Vertreter: Zenz Patentanwälte Partnerschaft mbB

(56) Entgegenhaltungen:
- EP-A1- 3 530 601
- WO-A1-2019/183141
- DE-A1- 10 225 332
- DE-A1- 102004 001 198
- DE-A1- 102014 111 394
- DE-B3- 102015 118 832
- US-B1- 9 466 046

## Beschreibung

Die vorliegende Erfindung betrifft ein Verfahren zum Betreiben einer Kommissioniervorrichtung für Arzneimittel sowie eine Kommissioniervorrichtung, die entsprechend dem erfindungsgemäßen Verfahren betrieben werden kann.

Bei zum Beispiel aus der DE 102 25 332 A1, welche den Oberbegriff der Ansprüche 1 sowie 11 offenbart, bekannten Kommissioniervorrichtungen für Arzneimittel ist regelmäßig mindestens ein Bediengerät verbaut, dessen Greifer an entsprechenden Führungen in der Längsachse (X-Richtung bzw. X-Achse) und der Höhe (Z-Richtung bzw. Z-Achse) verfahrbar ist, um unterschiedliche Regalfächer in der Kommissioniervorrichtung sowie eine Einlagerungseinrichtung und eine Entladeeinrichtung erreichen zu können. Moderne Kommissioniervorrichtungen für Arzneimittel arbeiten weitgehend automatisch, das heißt ein Betreten des Innenraumes der Kommissioniervorrichtung ist nur dann notwendig, wenn beispielsweise eine Reinigung von Teilen innerhalb der Kommissioniervorrichtung notwendig ist oder eine Fehlfunktion das Eingreifen eines Bedieners erfordert, beispielsweise wenn eine Lagerung oder Entnahme einer Arzneimittelpackung nicht ordnungsgemäß durchgeführt werden konnte. Bei jedem Eingriff durch einen Bediener ist es möglich, dass dieser einen Gegenstand, beispielsweise eine Leiter zum Erreichen von höher gelegenen Abschnitten der Kommissioniervorrichtung, unbeabsichtigt in der Kommissioniervorrichtung zurücklässt. In Abhängigkeit von der Größe und der Positionierung dieses unbeabsichtigt zurückgelassenen Gegenstandes kann dieser ein Hindernis für das Bediengerät darstellen. Im Extremfall kann eine übliche Bewegung des Bediengerätes zu einer Kollision mit dem zurückgelassenen Gegenstand führen, was zu einer Beschädigung des Bediengerätes führen kann. Eine Kollision kann auch dazu führen, dass Arzneimittelpackungen auf einer oder mehreren Lagerflächen derart durcheinandergebracht werden, dass eine manuelle Auslagerung notwendig ist. In Abhängigkeit von dem Hindernis und der Kollision ist es auch möglich, dass ein aufgrund der Kollision umkippendes Hindernis mehrere Regalflächen vollständig zerstört. In jedem Fall bedingt eine Kollision zwischen dem Bediengerät und einem in der Kommissioniervorrichtung zurückgelassenen Hindernis einen Eingriff durch einen Bediener, was wiederum zu Stillstandszeiten der Kommissioniervorrichtung führt.

Es ist daher Aufgabe der Erfindung, ein Verfahren zum Betreiben einer Kommissioniervorrichtung bereitzustellen, bei welchem Stillstandszeiten bedingt durch Kollisionen des Bediengerätes mit Hindernissen vermieden werden. Es ist ferner Aufgabe der vorliegenden Erfindung, eine Kommissioniervorrichtung bereitzustellen, mit welcher ein entsprechendes Verfahren durchführbar ist.

Die Aufgabe wird gelöst durch ein Verfahren zum Betreiben einer Kommissioniervorrichtung für Arzneimittel gemäß Patentanspruch 1. Die zur Durchführung des Verfahrens verwendete Kommissioniervorrichtung umfasst eine Mehrzahl von Lagerplätzen für Arzneimittelflaschen und/oder -packungen, ein vor den Lagerplätzen horizontal in einer X-Richtung bzw. X-Achse und vertikal in einer Z-Richtung bzw. Z-Achse in einem Bewegungsraum verfahrbares, einen Greifer aufweisendes Bediengerät, zumindest eine Einlagerungseinrichtung, mit welcher Arzneimittelflaschen und/oder -packungen in die Kommissioniervorrichtung bewegt werden können und von welcher der Greifer Arzneimittelflaschen und/oder -packungen entnehmen kann, zumindest eine Identifiziereinrichtung zum Identifizieren von Arzneimittelflaschen und/oder -packungen, eine Entladeeinrichtung, an welche Arzneimittelflaschen und/oder -packungen von dem Bediengerät zum Auslagern aus der Kommissioniervorrichtung übergeben werden können, und eine mit dem Bediengerät und der Identifiziereinrichtung gekoppelte Steuereinrichtung. Darüber hinaus umfasst die Kommissioniervorrichtung eine mit der Steuereinrichtung gekoppelte optische Erfassungseinrichtung, die derart in der Kommissioniervorrichtung angeordnet ist, dass diese ein Bild des Bewegungsraumes erstellen kann.

Bei dem erfindungsgemäßen Verfahren wird in einem Schritt a) nach einem vorgegebenen Ereignis, bei welchem es sich beispielsweise um eine routinemäßige Reinigung des Innenraumes der Kommissioniervorrichtung handeln kann, mit der zumindest einen optischen Erfassungseinrichtung ein Bild des von der optischen Erfassungseinrichtung erfassbaren Abschnitts des Bewegungsraumes erstellt. Ob dieses Bild den gesamten Bewegungsraum umfasst hängt u. a. von der genauen Platzierung der optischen Erfassungseinrichtung sowie der Größe des Bewegungsraums ab. Wenn die optische Erfassungseinrichtung beispielsweise an einer Stirnseite der Kommissioniervorrichtung angeordnet ist und die Erstreckung entlang der X-Achse nicht zu groß ist, ist es denkbar, dass mit einer optischen Erfassungseinrichtung der gesamte Bewegungsraum in einem Bild erfasst werden kann. Es ist aber auch denkbar, dass die optische Erfassungseinrichtung in bzw. an beispielsweise dem Greifer des Bediengeräts angeordnet ist. In einem solchen Fall ist es u. a. von der Positionierung des Bediengeräts innerhalb der Kommissioniervorrichtung abhängig, ob ein Bild ausreicht. Wenn das Bediengerät beispielsweise im Hinblick auf die X-Achse in der Mitte der Kommissioniervorrichtung angeordnet ist, kann es notwendig sein, dass ein Bild "links" und ein Bild "rechts" von dem Bediengerät erfasst werden muss. In einem solchen Fall ist es bei sehr langen Kommissioniervorrichtungen auch denkbar, dass mehrere Bilder erstellt werden müssen bis der gesamte Bewegungsbereich überprüft ist. Dazu wird dann das Bediengerät immer nur so weit in X-Richtung verfahren wie der Bewegungsraum erfasst und als frei erkannt wurde.

In einem nachfolgenden Verfahrensschritt b) werden vorgegebene Bereiche des Bildes des Bewegungsraumes mit entsprechenden Bereichen eines Referenz-Bildes verglichen. Bei den "vorgegebenen Bereichen" kann es sich um das vollständige aufgenommene Bild handeln. Da das Bild des Bewegungsraumes jedoch zwangsläufig auch Bereiche umfasst, die für eine nachfolgende Auswertung irrelevant sind (Bereiche der Lagerplätze für Arzneimittelflaschen und/oder -packungen), ist es auch denkbar, dass lediglich Teilbereiche des Bildes des Bewegungsraumes mit entsprechenden Teilbereichen des Referenz-Bildes verglichen werden. In einem Verfahrensschritt c) wird anhand des Vergleiches der vorgegebenen Bereiche des Bildes und des Referenz-Bildes ermittelt, ob ein Hindernis in dem erfassten Abschnitt des Bewegungsraums vorhanden ist. Wie genau die Ermittlung eines Hindernisses durchgeführt wird, ist für die vorliegende Erfindung nicht relevant; wesentlich ist lediglich, dass anhand des Vergleiches ermittelt wird, ob ein Hindernis vorhanden ist, das ggf. einer Bewegung des Bediengerätes entgegenstehen könnte. Dazu gibt es verschiedene, dem Fachmann bekannte Methoden, von denen eine bei der detaillierten Beschreibung der Erfindung dargelegt wird. In einem abschließenden Verfahrensschritt d) werden basierend auf dem Ermitteln des Vorhandenseins eines Hindernisses in dem erfassten Abschnitt des Bewegungsraums entsprechende Signale bereitgestellt. In einem einfachsten Fall bedingen diese Signale, dass ein Verfahren des Bediengerätes unterbunden wird, und zwar unabhängig von der Positionierung und Größe des Hindernisses. Darüber hinaus ist es denkbar, dass in einem solchen Fall ein Bediener auf die Anwesenheit eines Hindernisses hingewiesen wird, was beispielsweise optisch mittels einer Anzeigeeinrichtung oder akustisch durchgeführt werden kann.

In der einfachsten Form des erfindungsgemäßen Verfahrens wird bei Detektion eines Hindernisses das Verfahren des Bediengerätes schlichtweg blockiert. Bei einer bevorzugten Ausführungsform des erfindungsgemäßen Verfahrens wird in dem Verfahrensschritt d) des erfindungsgemäßen Verfahrens nach Durchführen des Vergleiches von vorgegebenen Bereichen des Bildes und des Referenz-Bildes die Position und die Größe eines Hindernisses in dem erfassten Abschnitt des Bewegungsraums ermittelt. Die Kenntnis dieser Informationen ermöglicht es, abschätzen zu können, ob ein Blockieren des Verfahrens des Bediengerätes überhaupt notwendig ist. In Abhängigkeit von den verwendeten Verarbeitungsalgorithmen der Bildanalysesoftware kann es dazu notwendig sein, in der Kommissioniervorrichtung einige Referenzpunkte anzubringen, anhand welcher Größe und Position bestimmbar sind.

Basierend auf den vorgenannten Informationen ist es bevorzugt, dass anhand der ermittelten Position und Größe des Hindernisses bestimmt wird, ob das Hindernis von dem Bediengerät umfahren werden kann. Sofern dies der Fall ist, kann der Benutzer vor die Wahl gestellt werden, ein Hindernis umgehend zu entfernen oder dies erst zu einem späteren Zeitpunkt zu tun und die Kommissioniervorrichtung solange trotz des Hindernisses weiter zu betreiben. Das Bediengerät wird dann selbstverständlich derart angesteuert, dass das vorhandene Hindernis umfahren und eine Kollision vermieden wird.

Das Hindernis kann derart positioniert und/oder ausgebildet sein, dass keine Lagerfläche der Kommissioniervorrichtung betroffen ist, das heißt trotz des vorhandenen Hindernisses auf sämtliche Arzneimittelflaschen und/oder -packungen zugegriffen werden kann. Beispielsweise ist es denkbar, dass das Hindernis lediglich einen Teilbereich der Entladeeinrichtung blockiert, was ggf. ein Einlagern von Arzneimittelflaschen und/oder -packungen verhindert, nicht jedoch ein Auslagern. In einem solchen Fall ist es durchaus denkbar, dass die Kommissioniervorrichtung in der Öffnungszeit einer Apotheke weiter betrieben wird und gleich im Anschluss an die reguläre Öffnungszeit das Hindernis entfernt wird. Bei einer bevorzugten Ausführungsform ist es vorgesehen, dass bei Umfahrbarkeit eines Hindernisses der von einem Hindernis blockierte Bereich als nicht erreichbar bzw. nicht zu befahren markiert wird. Dies ist insbesondere dann sinnvoll, wenn in diesem Bereich Arzneimittelpackungen gelagert sind. Bei Kenntnis, welche Lagerbereiche in der Kommissioniervorrichtung aufgrund des Hindernisses nicht erreicht werden können, lässt sich bei Vorliegen eines Auslagerungswunsches sofort feststellen, ob ggf. aufgrund des Hindernisses eine ansonsten auslagerbare Arzneimittelpackung nicht ausgelagert werden kann.

Sofern ein Hindernis detektiert ist, muss dieses unmittelbar oder ggf. zu einem späteren Zeitpunkt von einem Benutzer entfernt werden. Dies gilt jedoch nur für den Fall, dass das Hindernis nicht beispielsweise durch das Bediengerät selbst aus dem Weg geschafft werden kann. Bei einer bevorzugten Ausführungsform des erfindungsgemäßen Verfahrens ist es daher vorgesehen, dass anhand der ermittelten Position und Größe des Hindernisses bestimmt wird, ob ein Hindernis mit dem Bediengerät bewegbar ist. Ist dies der Fall, kann versucht werden das Hindernis mit dem Greifer des Bediengerätes zu greifen und an einem vorgegebenen Ort abzustellen, wobei es sich beispielsweise um die Entladungseinrichtung handeln kann. So ist es beispielsweise denkbar, dass wenn lediglich ein Hindernis von geringer Größe auf beispielsweise der X-Führung des Bediengerätes positioniert ist, dieses mit dem Greifer und ohne ein Zutun eines Bedieners entfernt wird.

Um zu ermitteln, ob ein Hindernis in dem Bewegungsraum innerhalb der Kommissioniervorrichtung vorhanden ist, wird ein Vergleich von vorgegebenen Bereichen des Bildes und des Referenz-Bildes durchgeführt. Um zu vermeiden, dass der Greifer des Bediengerätes bei der Aufnahme des Bildes ein ggf. vorhandenes Hindernis verdeckt, ist es bei einer bevorzugten Ausführungsform des Verfahrens vorgesehen, dass der Greifer des Bediengerätes vor dem Erstellen des Bildes entlang der Z-Achse in eine maximale Z-Position verfahren wird.

In Abhängigkeit von der Ausdehnung der Kommissioniervorrichtung in Längsrichtung, also entlang der **X-**Achse, kann es ausreichend sein, dass lediglich eine optische Erfassungseinrichtung verwendet wird. Bei Kommissioniervorrichtungen die eine bestimmte Ausdehnung in **X-**Richtung überschreiten, kann es jedoch notwendig sein, dass mehrere optische Erfassungseinrichtungen verwendet werden. In einem solchen Fall ist es bevorzugt, dass das Bild und das Referenz-Bild aus mehreren Einzelbildern zusammengesetzt werden und die Ermittlung des Hindernisses dann auf der Basis der zusammengesetzten Bilder erfolgt. Alternativ ist es denkbar, dass ein Vergleichen des Bildes und des Referenz-Bildes auf der Basis von Teilbildern durchgeführt wird. Sofern die optische Erfassungseinrichtung in bzw. an dem Greifer angeordnet bzw. realisiert ist, beispielsweise durch eine 2D- oder 3D-Kamera, kann es notwendig sein, das Verfahren mehrfach zu wiederholen, wobei bei jeder Wiederholung ein Abschnitt des Bewegungsraums erfasst und untersucht wird, das erfindungsgemäße Verfahren also pro Abschnitt durchgeführt wird.

Die Aufgabe wird ferner gelöst durch eine Kommissioniervorrichtung für Arzneimittel nach Anspruch 11. Die erfindungsgemäße Kommissioniervorrichtung umfasst eine Mehrzahl von Lagerplätzen für Arzneimittelflaschen und/oder -packungen, wobei die Lagerplätze in Abhängigkeit von den einzulagernden Arzneimitteln (Packung oder Flasche) durch horizontale Regalflächen mit ggf. Aufnahmen für Flaschen bereitgestellt sein können. Die Kommissioniervorrichtung umfasst ferner ein vor den Lagerplätzen horizontal in einer X-Richtung (X-Achse) und vertikal in einer Z-Richtung (Z-Achse) in einem Bewegungsraum verfahrbares, einen Greifer aufweisendes Bediengerät, zumindest eine Einlagerungseinrichtung, mit welcher Arzneimittelflaschen und/oder -packungen in die Kommissioniervorrichtung bewegt werden können und von welcher der Greifer Arzneimittelflaschen und/oder -packungen entnehmen kann, zumindest eine Identifiziereinrichtung zum Identifizieren von Arzneimittelflaschen und/oder -packungen, eine Entladeeinrichtung, an welche Arzneimittelflaschen und/oder -packungen von dem Bediengerät zum Auslagern aus der Kommissioniervorrichtung übergeben werden können und zumindest eine optische Erfassungseinrichtung, die derart in der Kommissioniervorrichtung angeordnet ist, dass diese ein Gesamtbild des Bewegungsraums erstellen kann, sowie eine mit dem Bediengerät, der Identifiziereinrichtung und der optischen Erfassungseinrichtung gekoppelte Steuereinrichtung, die ausgebildet ist, die Verfahrensschritte des oben beschriebenen erfindungsgemäßen Verfahrens auszuführen.

Bei einer bevorzugten Ausführungsform der Kommissioniervorrichtung ist es vorgesehen, dass die optische Erfassungseinrichtung ein Bauteil des Greifers des Bediengeräts ist. Bei dieser Ausführungsform wird also ein Bauteil genutzt, das üblicherweise jeder Greifer aufweist, um das erfindungsgemäße Verfahren zu realisieren. Die zusätzlichen Kosten für die Umsetzung des Verfahrens sind in diesem Fall auf notwendige Anpassungen der Software beschränkt, da keine neuen Bauteile in eine bereits vorhandene Kommissioniervorrichtung zu integrieren sind.

Im Nachfolgenden wird eine bevorzugte Ausführungsform des erfindungsgemäßen Verfahrens sowie eine bevorzugte Ausführungsform der erfindungsgemäßen Kommissioniervorrichtung unter Bezugnahme auf die Zeichnung beschrieben, in welcher
Figuren 1a und 1b Schrägansichten einer Ausführungsform der erfindungsgemäßen Kommissioniervorrichtung zeigen,
Figur 2 eine Draufsicht auf die Kommissioniervorrichtung zeigt,
Figur 3 eine seitliche Schnittansicht zeigt, bei welcher eine Regalreihe fortgelassen ist,
Figuren 4 und 5 den Figuren 2 und 3 entsprechende Ansichten zeigen, wobei jeweils ein Hindernis in dem Bewegungsraum der Kommissioniervorrichtung angeordnet ist,
Figuren 6a und 6b Gesamtbilder des Bewegungsraumes mit an verschiedenen Positionen angeordneten Hindernissen zeigen, und
Figuren 7a - 7d schematisch den Vergleich zwischen Gesamtbild und Referenz-Gesamtbild veranschaulichen, und
Figur 8 ein Ablaufschema einer bevorzugten Ausführungsform des erfindungsgemäßen Verfahrens veranschaulicht.

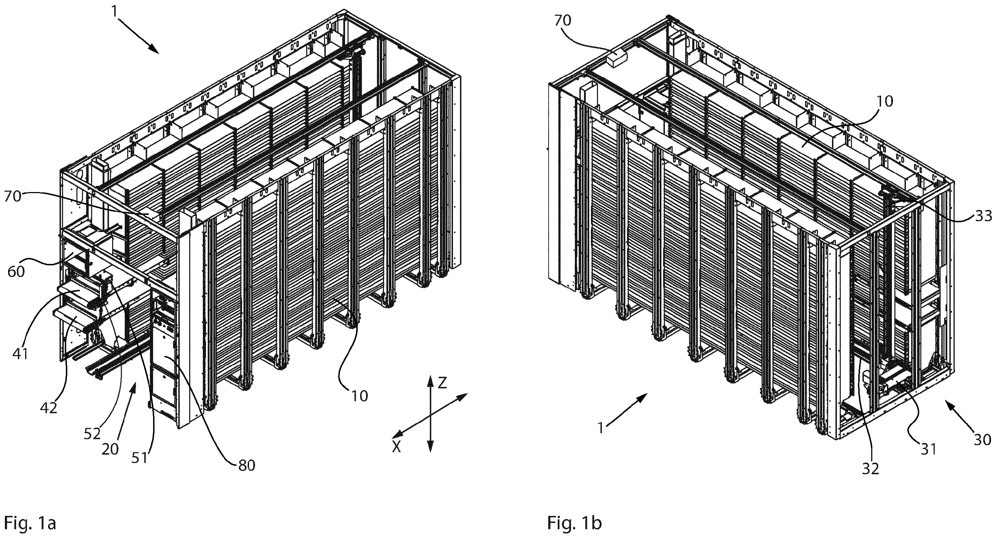

Die Figuren 1a und 1b zeigen Schrägansichten einer Ausführungsform der erfindungsgemäßen Kommissioniervorrichtung 1 für Arzneimittelpackungen, wobei bei der gezeigten Ausführungsform die Außenverkleidung fortgelassen ist. Bei der gezeigten Ausführungsform umfasst die Kommissioniervorrichtung eine Mehrzahl von Regalböden 10, die zwei parallele Regalreihen bilden, zwischen denen ein Bewegungsraum 20 ausgebildet ist, in welchem ein einen Greifer 31 aufweisendes Bediengerät 30 horizontal in einer X-Richtung und vertikal in einer Z-Richtung verfahrbar ist. Die Kommissioniervorrichtung umfasst dazu eine Horizontal- bzw. X-Führung 32 sowie eine Vertikal- bzw. Z-Führung 33. Die Kommissioniervorrichtung umfasst ferner zwei Einlagerungseinrichtungen 41, 42, die bei der gezeigten Ausführungsform als Förderbänder ausgebildet sind. Bei der in Figur 1a dargestellten Einlagerungsstirnseite reichen diese Einlagerungseinrichtungen mit einem Abschnitt über die eigentliche Einlagerungsstirnseite der Kommissioniervorrichtung hinaus, und in diesen Abschnitten kann ein Bediener einzulagernde Arzneimittelpackungen auflegen. Oberhalb jeder Einlagerungseinrichtung ist im Einlagerungsstirnbereich der Kommissioniervorrichtung eine Identifiziereinrichtung 51, 52 angeordnet, mit welcher die einzulagernden Arzneimittelpackungen identifiziert und ggf. vermessen werden. Die Kommissioniervorrichtung umfasst ferner eine Entladeeinrichtung 60, an welche der Greifer 31 des Bediengerätes 30 Arzneimittelpackungen übergeben kann. Das Bediengerät 30 sowie die Identifiziereinrichtungen 51, 52 sind mit einer Steuereinrichtung 80 gekoppelt, die bei der gezeigten Ausführungsform ebenfalls im Einlagerungsstirnbereich (siehe Figur 1a) angeordnet ist. Die Kommissioniervorrichtung weist erfindungsgemäß eine optische Erfassungseinrichtung 70 auf, die derart in der Kommissioniervorrichtung angeordnet ist, dass diese ein Gesamtbild des Bewegungsraumes 20 erstellen kann. Bei der gezeigten Ausführungsform ist die optische Erfassungseinrichtung ebenfalls bei der Einlagerungsstirnseite angeordnet. Alternativ kann die optische Erfassungseinrichtung auch bei der in Figur 1b gezeigten rückseitigen Stirnseite oder mittig über dem Bewegungsraum angeordnet sein, wobei die ideale Anordnung der optischen Erfassungseinrichtung von der Art der verwendeten Erfassungseinrichtung und der Gestaltung des Bewegungsraums abhängig ist.

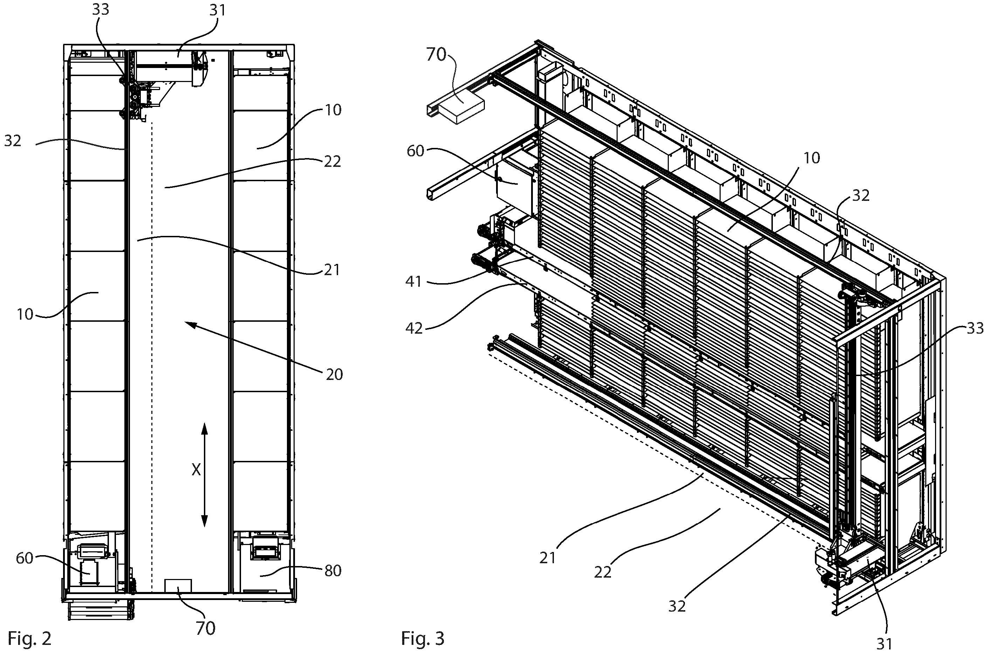

Die Figur 2 zeigt eine Draufsicht auf die bevorzugte Ausführungsform der erfindungsgemäßen Kommissioniervorrichtung, wobei bei dieser Draufsicht insbesondere der zwischen den Regalreihen ausgebildete Bewegungsraum 20 für das Bediengerät 30 erkennbar ist. Der Bewegungsraum 20 ist in Figur 2 mittels einer gestrichelten Linie in einen linken Bewegungsraumabschnitt 21 und einen rechten Bewegungsraumabschnitt 22 unterteilt. Aufgrund der Anordnung der horizontalen X-Führung 32 handelt es sich bei dem Bewegungsraumabschnitt 21 um einen "kritischen" Bewegungsraumabschnitt, in welchem ein Hindernis in jedem Fall zu einer Kollision mit einem in X-Richtung verfahrenen Bediengerät führt, und zwar aufgrund der Tatsache, dass bei einer Bewegung in X-Richtung stets die Z-Führung 33 in X-Richtung verfahren wird. Bei dem Bewegungsraumabschnitt 22 handelt es sich um einen "nicht kritischen" Bewegungsraumabschnitt, da in diesem Bewegungsraumabschnitt des Bewegungsraumes 20 angeordnete Hindernisse ggf. durch eine entsprechende Bewegung des Greifers des Bediengerätes umfahren werden können.

Figur 3 zeigt eine Schnittansicht, bei der insbesondere die untere X-Führung 32 des Bediengerätes 30 erkennbar ist. Ferner ist erkennbar, warum der Bewegungsraumabschnitt 21 als "kritischer" Bewegungsbereich definiert ist. Selbst wenn der Greifer 31 zum Bewegen eines Hindernisses in Z-Richtung nach oben verfahren wird, bedingt ein Hindernis in dem kritischen Bewegungsraumabschnitt 21 aufgrund des Verfahrens der Z-Führung in X-Richtung eine Kollision zwischen dem Hindernis und dem Bediengerät. Bei dem Greifer 31 des Bediengeräts ist ebenfalls eine optische Erfassungseinrichtung 34 vorgesehen, beispielsweise eine 2D- oder 3D-Kamera, die zum üblichen Betrieb des Greifers regelmäßig notwendig ist. Darüber hinaus kann diese optische Erfassungseinrichtung bei einer alternativen Ausführungsform zum Erfassen eines Bildes des Bewegungsraums genutzt werden, wie dies weiter oben bereits beschrieben ist.

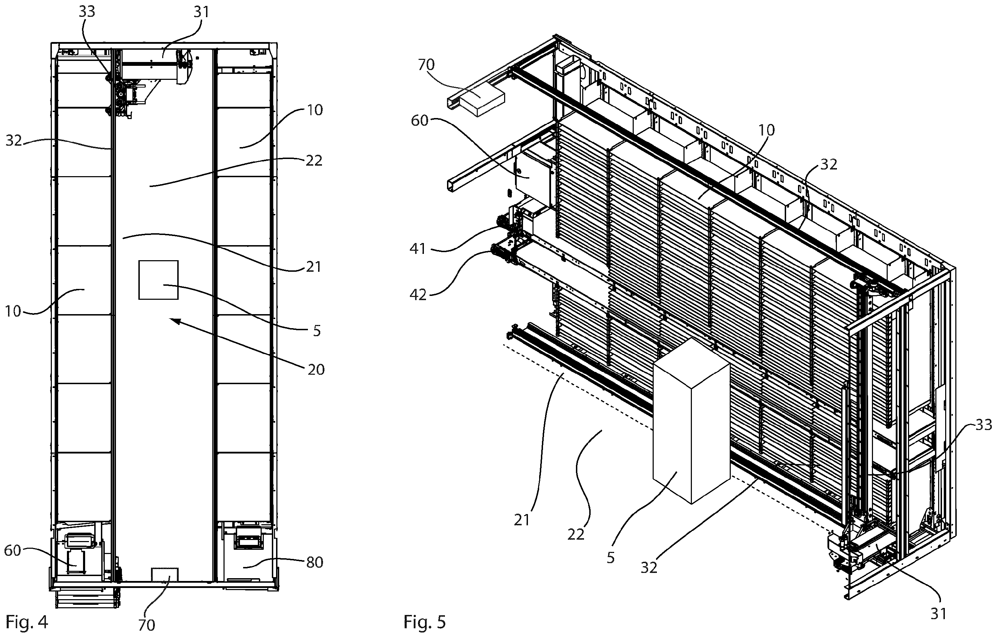

Figuren 4 und 5 zeigen die Ansichten der Figuren 2 und 3, wobei bei Figuren 4 und 5 ein Hindernis 5 in dem nicht kritischen Bewegungsraumabschnitt 22 angeordnet ist. Aufgrund der Ausgestaltung des Bediengerätes mit Anordnung der X-Führung bei einer Seite des Bewegungsraumes ist theoretisch ein Umfahren des Hindernisses mit dem Bediengerät möglich. Ob bei Ermittlung eines Hindernisses dieses umfahren wird oder gleich ein Verfahren des Bediengerätes grundsätzlich untersagt wird, hängt zum einen davon ab, ob die Position und die Größe des Hindernisses ermittelt werden kann, und zum anderen von den Vorgaben des Bedieners.

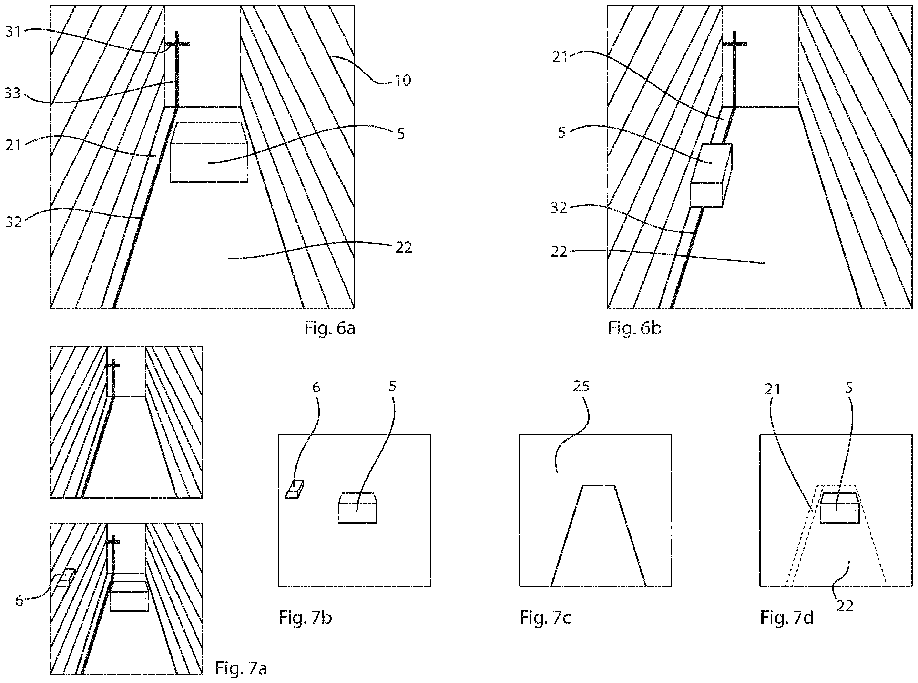

Figuren 6a und 6b zeigen schematisch zwei Bilder des Bewegungsraumes 20 mit unterschiedlich angeordneten Hindernissen 5, wobei die dargestellten Bilder jeweils den gesamten Bewegungsraum darstellen sollen. Bei der in Figur 6a gezeigten Situation ist ein Hindernis 5 in dem "unkritischen" Bewegungsraumabschnitt 22 angeordnet. Wie dies anhand der schematisch dargestellten X- und Z-Führung erkennbar ist, ist es bei der in Figur 6a gezeigten Situation möglich, dass das Bediengerät bei entsprechender Ausrichtung des Greifers 31 das Hindernis 5 ohne Kollision umfährt. Eine entsprechende Verfahrensführung ist jedoch nur dann möglich, wenn die genaue Position und Größe des Hindernisses 5 ermittelbar sind, was unter anderem von der Größe selbst und dem Kontrast Hindernis/Hintergrund abhängig ist. Bei der in Figur 6b gezeigten Situation, bei welcher das Hindernis 5 die X-Führung 32 behindert, also in dem kritischen Bewegungsraumabschnitt 21 angeordnet ist, ist ein Weiterbetrieb der Kommissioniervorrichtung zumindest dann unmöglich, wenn die gesamte sich in X-Richtung erstreckende Länge der Kommissioniervorrichtung genutzt werden soll. Wird ein solcher Fall detektiert, ist das Eingreifen bei oben genannten Voraussetzungen durch einen Bediener unumgänglich.

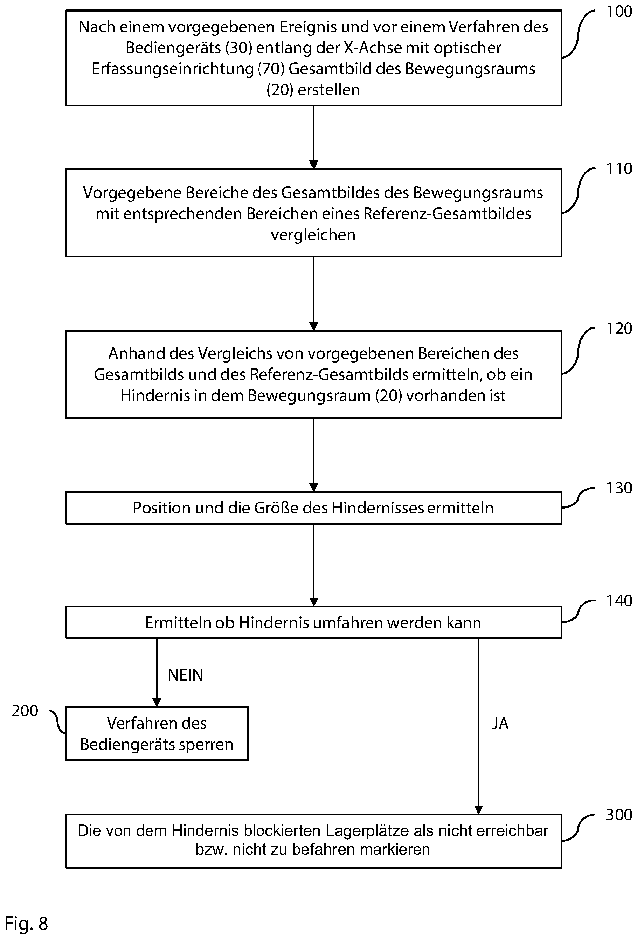

Im nachfolgenden wird unter Bezugnahme auf die Figuren 7a - 7d und Figur 8 eine bevorzugte Ausführungsform des erfindungsgemäßen Verfahrens beschrieben, wobei die Figuren 7a - 7d schematisch das Ermitteln eines Hindernisses auf der Basis von Bild und Referenz-Bild veranschaulichen. Auch in diesem Fall ist der gesamte Bewegungsraum von dem Bild und dem Referenz-Bild erfasst, die optische Erfassungseinrichtung ist bei der dem Bediengerät gegenüberliegenden Stirnseite der Kommissioniervorrichtung angeordnet. Zunächst wird in einem Schritt 100 bei Vorliegen eines vorgegebenen Ereignisses, beispielsweise einer routinemäßigen Reinigung des Innenraums der Kommissioniervorrichtung, und bei dieser Ausführungsform vor dem Verfahren des Bediengerätes 30 entlang der X-Achse, mit der (nicht dargestellten) optischen Erfassungseinrichtung 70 ein Bild des Bewegungsraums 20 erstellt. Zwei schematische Ansichten eines solchen Bildes sind in den Figuren 6a und 6b gezeigt, wobei bei Figur 6a das Hindernis in dem unkritischen Bewegungsraumabschnitt 22 und bei Figur 6b in dem kritischen Bewegungsraumabschnitt 21 angeordnet ist. Anschließend werden in einem Schritt 110 vorgegebene Bereiche des Bildes des Bewegungsraums mit entsprechenden Bereichen eines Referenz-Bildes verglichen. In Figur 7a sind zur Erläuterung des Ermittelns eines Hindernisses im oberen Abschnitt ein Referenz-Bild und im unteren Abschnitt ein von der optischen Erfassungseinrichtung aufgenommenes Bild schematisch veranschaulicht. Aufgrund der Geometrie des Innenraumes der Kommissioniervorrichtung umfasst das Bild des Bewegungsraumes stets nicht nur den Bewegungsraum selbst, sondern auch Abschnitte der beiden gegenüberliegenden Regalreihen, die in Figur 7a schematisch angedeutet sind.

Entsprechend einer bevorzugten Ausführungsform wird zur Ermittlung eines Hindernisses wie folgt vorgegangen. Zunächst werden das Referenz-Bild und das aktuelle Bild voneinander abgezogen, so dass lediglich die Objekte verbleiben, die in dem Bild, nicht aber in dem Referenz-Bild vorhanden sind. Bei dem in Figur 7a gezeigten Beispiel sind das eine Arzneimittelpackung 6 sowie ein Hindernis 5; das Ergebnis der Differenzbildung ist in Figur 7b schematisch veranschaulicht.

Basierend auf dem Vergleich des Bildes und des Referenz-Bildes wird nun ermittelt, ob ein Hindernis in dem Bewegungsraum vorhanden ist (Schritt 120). Da das Bild (sowie das Referenz-Bild) stets auch Bereiche umfasst, die nicht den Bewegungsraum selbst betreffen, sondern Teile der seitlichen Regallager, wird dazu das Ergebnis der Subtraktion zwischen Bild und Referenz-Bild mit einer in Figur 7c dargestellten Maske 25 kombiniert, die solche Bereiche des Ergebnisses der Differenzbildung ausblendet, die nicht den Bewegungsraum selbst betreffen. Das Ergebnis dieser Kombination des Differenzbildes mit der Maske ist in Figur 7d dargestellt; es verbleibt lediglich das Hindernis 5, welches nun mit üblichen bekannten Bearbeitungsverfahren weiterbearbeitet wird. Dazu wird das in Figur 7d angedeutete Bild üblicherweise in ein Schwarzweißbild überführt (binarisiert) und einer anschließenden Blobanalyse unterzogen. All jene Bereiche, die von ihrer Größe her eine bestimmte vorgegebene Größe überschreiten, werden dann als Hindernis klassifiziert und, falls möglich und gewünscht, deren Position und Größe an die Steuereinrichtung weitergegeben. In Abhängigkeit von der genauen Verfahrensführung kann bei Anwesenheit eines Hindernisses jegliche Bewegung des Bediengerätes untersagt werden, alternativ ist es möglich, ein Umfahren des Hindernisses vorzugeben oder, bei optimal positionierten kleinen Hindernissen, ein Verräumen der Hindernisse mit dem Greifer des Bediengerätes zu initiieren.

Bei der in Figur 8 dargestellten Ausführungsform wird in einem Schritt 130 die Größe und die Position des Hindernisses ermittelt. Dazu kann es notwendig sein, in der Kommissioniervorrichtung Referenzpunkte vorzugeben und/oder die Steuereinrichtung anhand einer Mehrzahl von Referenz-Gesamtbildern in Bezug auf die Ermittlung der Größe und der Position anzulernen. Alternativ oder zusätzlich kann es auch vorgesehen sein, den Boden des Bewegungsbereichs und/oder die beiden Bewegungsraumabschnitte mit speziellen Mustern und/oder Kennzahlen zu versehen. In Kenntnis des Befestigungsortes der optischen Erfassungseinrichtung kann dann in einem günstigen Fall Größe und Position ermittelt werden.

Basierend auf der Kenntnis der Größe und der Position des Hindernisses wird in einem Schritt 140 anhand der baulichen Gestaltung des Bewegungsraums ermittelt, ob das Hindernis umfahren werden kann. Ist dies nicht der Fall, beispielsweise weil das Hindernis zumindest zum Teil in dem kritischen Bewegungsraumabschnitt angeordnet ist, wird das Bediengerät gesperrt, ein weiteres Verfahren also so lange unterbunden (Schritt 200), bis ein Bediener das Entfernen des Hindernisses anzeigt.

Sofern das Umfahren möglich (und gewollt) ist, werden diejenigen Lagerplätze, die aufgrund des Hindernisses nicht erreicht werden können, entsprechend markiert. Für den dann möglichen weiteren Betrieb der Kommissioniervorrichtung heißt das, dass die bei diesen Lagerplätzen lagernden Arzneimittelpackungen nicht ausgelagert werden können.

Bei alternativen Ausführungsformen kann es erforderlich sein, das oben beschriebene Verfahren für mehrere Abschnitte der Kommissioniervorrichtung zu wiederholen (wenn die optische Erfassungseinrichtung bei bzw. an dem Greifer angeordnet bzw. genutzt wird und nicht der gesamte Bewegungsraum mit einem Bild erfasst werden kann) oder das Bild aus mehreren Teilbildern zusammenzusetzen (wenn mehrere optische Erfassungseinrichtungen verwendet werden).

## Patentansprüche

1. Verfahren zum Betreiben einer Kommissioniervorrichtung für Arzneimittel, aufweisend
eine Mehrzahl von Lagerplätzen für Arzneimittelflaschen und/oder -packungen (6),
ein vor den Lagerplätzen horizontal in einer X-Richtung (X-Achse) und vertikal in einer Z-Richtung (Z-Achse) in einem Bewegungsraum (20) verfahrbares, einen Greifer (31) aufweisendes Bediengerät (30),
zumindest eine Einlagerungseinrichtung (41, 42), mit welcher Arzneimittelflaschen und/oder -packungen (6) in die Kommissioniervorrichtung (1) bewegt werden können und von welcher der Greifer (31) Arzneimittelflaschen und/oder -packungen (6) entnehmen kann,
zumindest eine Identifiziereinrichtung (51, 52) zum Identifizieren von Arzneimittelflaschen und/oder -packungen (6),
eine Entladeeinrichtung (60), an welche Arzneimittelflaschen und/oder -packungen (6) von dem Bediengerät zum Auslagern aus der Kommissioniervorrichtung (1) übergeben werden können,
zumindest eine optische Erfassungseinrichtung (34, 70), die derart in der Kommissioniervorrichtung (1) angeordnet ist, dass diese ein Bild des Bewegungsraums (20) erstellen kann, und
eine mit dem Bediengerät (30), der Identifiziereinrichtung (51, 52) und der optischen Erfassungseinrichtung (34, 70) gekoppelte Steuereinrichtung (80), **dadurch gekennzeichnet, dass**
a) nach einem vorgegebenen Ereignis mit der zumindest einen optischen Erfassungseinrichtung (34, 70) ein Bild des von der optischen Erfassungseinrichtung erfassbaren Bewegungsraums (20) erstellt wird,
b) vorgegebene Bereiche des Bildes des Bewegungsraums (20) mit entsprechenden Bereichen eines Referenz-Bildes verglichen werden,
c) anhand des Vergleichs von vorgegebenen Bereichen des Bilds und des Referenz-Bilds ermittelt wird, ob ein Hindernis in dem erfassten Abschnitt des Bewegungsraums (20) vorhanden ist, und
d) basierend auf dem Ermitteln des Vorhandenseins eines Hindernisses entsprechende Signale bereitgestellt werden.

2. Verfahren zum Betreiben einer Kommissioniervorrichtung für Arzneimittel nach Anspruch 1, **dadurch gekennzeichnet, dass** in Schritt d) die Position und die Größe eines Hindernisses ermittelt werden.

3. Verfahren zum Betreiben einer Kommissioniervorrichtung für Arzneimittel nach Anspruch 2, **dadurch gekennzeichnet, dass** anhand der ermittelten Position und Größe des Hindernisses bestimmt wird, ob das Hindernis von dem Bediengerät umfahren werden kann.

4. Verfahren zum Betreiben einer Kommissioniervorrichtung für Arzneimittel nach Anspruch 3, **dadurch gekennzeichnet, dass** bei Umfahrbarkeit des Hindernisses der von einem Hindernis blockierte Lagerplatzbereich als nicht erreichbar bzw. nicht zu befahren markiert wird.

5. Verfahren zum Betreiben einer Kommissioniervorrichtung für Arzneimittel nach Anspruch 2, **dadurch gekennzeichnet, dass** anhand der ermittelten Position und Größe des Hindernisses bestimmt wird, ob das Hindernis mit dem Bediengerät bewegbar ist.

6. Verfahren zum Betreiben einer Kommissioniervorrichtung für Arzneimittel nach einem der Ansprüche 1 - 5, **dadurch gekennzeichnet, dass** der Greifer (31) des Bediengerätes (30) vor dem Erstellen des Gesamtbilds entlang der Z-Achse in eine maximale Z-Position verfahren wird.

7. Verfahren zum Betreiben einer Kommissioniervorrichtung für Arzneimittel nach einem der Ansprüche 1 - 6, **dadurch gekennzeichnet, dass** das Gesamtbild und das Referenz-Gesamtbild aus mehreren Einzelbildern zusammengesetzt werden.

8. Verfahren zum Betreiben einer Kommissioniervorrichtung für Arzneimittel nach einem der Ansprüche 1 - 6, **dadurch gekennzeichnet, dass** ein Vergleichen des Gesamtbildes und des Referenz-Gesamtbildes auf der Basis von Teilbildern durchgeführt wird.

9. Verfahren zum Betreiben einer Kommissioniervorrichtung für Arzneimittel nach Anspruch 1, **dadurch gekennzeichnet, dass** die bereitgestellten Signale ein Verfahren des Bediengerätes (30) temporär verhindern.

10. Verfahren zum Betreiben einer Kommissioniervorrichtung für Arzneimittel nach einem der Ansprüche 1 - 9, **dadurch gekennzeichnet, dass** das Vorhandensein eines Hindernisses einem Benutzer angezeigt wird.

11. Kommissioniervorrichtung (1) für Arzneimittel, aufweisend
eine Mehrzahl von Lagerplätzen für Arzneimittelflaschen und/oder -packungen (6),
ein vor den Lagerplätzen horizontal in einer X-Richtung (X-Achse) und vertikal in einer Z-Richtung (Z-Achse) in einem Bewegungsraum (20) verfahrbares, einen Greifer (31) aufweisendes Bediengerät (30),
zumindest eine Einlagerungseinrichtung (41, 42), mit welcher Arzneimittelflaschen und/oder -packungen (6) in die Kommissioniervorrichtung (1) bewegt werden können und von welcher der Greifer (31) Arzneimittelflaschen und/oder -packungen (6) entnehmen kann,
zumindest eine Identifiziereinrichtung (51, 52) zum Identifizieren von Arzneimittelflaschen und/oder -packungen (6),
eine Entladeeinrichtung (60), an welche Arzneimittelflaschen und/oder -packungen (6) von dem Bediengerät zum Auslagern aus der Kommissioniervorrichtung (1) übergeben werden können,
zumindest eine optische Erfassungseinrichtung (34, 70), die derart in der Kommissioniervorrichtung (1) angeordnet ist, dass diese ein Gesamtbild des Bewegungsraums (20) erstellen kann, und
eine mit dem Bediengerät (30), der Identifiziereinrichtung (51, 52) und der optischen Erfassungseinrichtung (34, 70) gekoppelte Steuereinrichtung (80), **dadurch gekennzeichnet, dass** die Steuereinrichtung dazu ausgebildet ist, die Verfahrensschritte entsprechend der Ansprüche 1 - 10 auszuführen.

12. Kommissioniervorrichtung (1) für Arzneimittel nach Anspruch 11, **dadurch gekennzeichnet, dass** die optische Erfassungseinrichtung (34) ein Bauteil des Greifers (31) des Bediengeräts (30) ist.

## Claims

1. A method for operating a picking device for medicaments, comprising
a plurality of storage spaces for medicament bottles and/or packages (6),
an operating device (30) having a gripper (31), wherein said operating device can be moved horizontally in an X-direction (X-axis) and vertically in a Z-direction (Z-axis) in front of the storage spaces in a movement space (20),
at least one storage device (41, 42) with which medicament bottles and/or packages (6) can be moved into the picking device (1) and from which the gripper (31) can take medicament bottles and/or packages (6),
at least one identification device (51, 52) for identifying medicament bottles and/or packages (6),
an unloading device (60), to which medicament bottles and/or packages (6) can be transferred by the operating device for removal from the picking device (1),
at least one optical detection device (34, 70), which is arranged in the picking device (1) such that it can create an image of the movement space (20), and
a control device (80) coupled to the operating device (30), the identification device (51, 52) and the optical detection device (34, 70), **characterized in that**
a) after a predetermined event an image of the movement space (20) detectable by an optical detection device is created with the at least one optical detection device (34, 70),
b) predefined areas of the image of the movement space (20) are compared with corresponding areas of a reference image,
c) it is determined, based on the comparison of predetermined areas of the image and the reference image, whether an obstacle is present in the detected portion of the movement space (20), and
d) based on the detection of the presence of an obstacle, corresponding signals are provided.

2. The method for operating a picking device for medicaments according to claim 1, **characterized in that** the position and the size of an obstacle are determined in step d).

3. The method for operating a picking device for medicaments according to claim 2, **characterized in that** it is determined on the basis of the determined position and size of the obstacle, whether the obstacle can be bypassed by the operating device.

4. The method for operating a picking device for medicaments according to claim 3, **characterized in that**, when the obstacle can be bypassed, the storage area blocked by an obstacle is marked as unreachable or not accessible.

5. The method for operating a picking device for medicaments according to claim 2, **characterized in that** it is determined on the basis of the determined position and size of the obstacle, whether the obstacle is movable with the operating device.

6. The method for operating a picking device for medicaments according to any of claims 1-5, **characterized in that** the gripper (31) of the operating device (30) is moved into a maximum Z-position along the Z-axis before creating the overall image.

7. The method for operating a picking device for medicaments according to any of claims 1-6, **characterized in that** the overall image and the overall reference image are composed of a plurality of individual images.

8. The method for operating a picking device for medicaments according to any of claims 1-6, **characterized in that** a comparison of the overall image and the overall reference image is performed on the basis of partial images.

9. The method for operating a picking device for medicaments according to claim 1, **characterized in that** the provided signals temporarily prevent a movement of the operating device (30).

10. The method for operating a picking device for medicaments according to any of claims 1-9, **characterized in that** the presence of an obstacle is displayed to a user.

11. A picking device (1) for medicaments, comprising
a plurality of storage spaces for medicament bottles and/or packages (6),
an operating device (30) having a gripper (31), wherein said operating device can be moved horizontally in an X-direction (X-axis) and vertically in a Z-direction (Z-axis) in front of the storage spaces in a movement space (20),
at least one storage device (41, 42) with which medicament bottles and/or packages (6) can be moved into the picking device (1) and from which the gripper (31) can take medicament bottles and/or packages (6),
at least one identification device (51, 52) for identifying medicament bottles and/or packages (6),
an unloading device (60), to which medicament bottles and/or packages (6) can be transferred by the operating device for removal from the picking device (1),
at least one optical detection device (34, 70) arranged in the picking device (1) such that it can create an overall image of the movement space (20), and
a control device (80) coupled to the operating device (30), the identification device (51, 52) and the optical detection device (34, 70), **characterized in that**
the control device is configured to carry out the method steps according to claims 1-10.

12. The picking device (1) for medicaments according to claim 11, **characterized in that** the optical detection device (34) is a component of the gripper (31) of the operating device (30).

## Revendications

1. Procédé d'exploitation d'un dispositif de préparation de commandes pour médicaments, présentant
une pluralité d'emplacements de stockage pour des bouteilles et/ou des emballages de médicaments (6),
un appareil de commande (30) présentant un préhenseur (31), déplaçable horizontalement dans une direction X (axe X) et verticalement dans une direction Z (axe Z) dans un espace de déplacement (20) devant les emplacements de stockage,
au moins un dispositif de stockage (41, 42), avec lequel des bouteilles et/ou des emballages de médicaments (6) peuvent être déplacés dans le dispositif de préparation de commandes (1) et duquel le préhenseur (31) peut prélever des bouteilles et/ou des emballages de médicaments (6),
au moins un dispositif d'identification (51, 52) pour identifier les bouteilles et/ou les emballages de médicaments (6),
un dispositif de déchargement (60) auquel des bouteilles et/ou des emballages de médicaments (6) peuvent être transférés par l'appareil de commande pour être déchargés du dispositif de préparation de commandes (1),
au moins un dispositif de détection optique (34, 70), qui est disposé dans le dispositif de préparation de commandes (1) de telle sorte que celui-ci peut établir une image de l'espace de déplacement (20), et
un dispositif de commande (80) couplé à l'appareil de commande (30), au dispositif d'identification (51, 52) et au dispositif de détection optique (34, 70), **caractérisé en ce que**
a) après un événement prédéfini, une image de l'espace de déplacement (20) pouvant être détecté par le dispositif de détection optique est établie avec le au moins un dispositif de détection optique (34, 70),
b) des zones prédéfinies de l'image de l'espace de déplacement (20) sont comparées à des zones correspondantes d'une image de référence,
c) à l'aide de la comparaison de zones prédéfinies de l'image et de l'image de référence, on détermine si un obstacle est présent dans la section détectée de l'espace de déplacement (20), et
d) sur la base de la détermination de la présence d'un obstacle, des signaux correspondants sont mis à disposition.

2. Procédé d'exploitation d'un dispositif de préparation de commandes pour médicaments selon la revendication 1, **caractérisé en ce que** la position et la taille d'un obstacle sont déterminées à l'étape d).

3. Procédé d'exploitation d'un dispositif de préparation de commandes pour médicaments selon la revendication 2, **caractérisé en ce qu'**à l'aide de la position et de la taille déterminées de l'obstacle, on détermine si l'obstacle peut être contourné par l'appareil de commande.

4. Procédé d'exploitation d'un dispositif de préparation de commandes pour médicaments selon la revendication 3, **caractérisé en ce que**, si l'obstacle peut être contourné, la zone de stockage bloquée par un obstacle est marquée comme non accessible ou non praticable.

5. Procédé d'exploitation d'un dispositif de préparation de commandes pour médicaments selon la revendication 2, **caractérisé en ce qu'**à l'aide de la position et de la taille déterminées de l'obstacle, on détermine si l'obstacle peut être déplacé avec l'appareil de commande.

6. Procédé d'exploitation d'un dispositif de préparation de commandes pour médicaments selon l'une des revendications 1 à 5, **caractérisé en ce que** le préhenseur (31) de l'appareil de commande (30) est déplacée le long de l'axe Z dans une position Z maximale avant la création de l'image globale.

7. Procédé d'exploitation d'un dispositif de préparation de commandes pour médicaments selon l'une des revendications 1 à 6, **caractérisé en ce que** l'image globale et l'image globale de référence sont composées de plusieurs images individuelles.

8. Procédé d'exploitation d'un dispositif de préparation de commandes pour médicaments selon l'une des revendications 1 à 6, **caractérisé en ce qu'**une comparaison de l'image globale et de l'image globale de référence est effectuée sur la base d'images partielles.

9. Procédé d'exploitation d'un dispositif de préparation de commandes pour médicaments selon la revendication 1, **caractérisé en ce que** les signaux mis à disposition empêchent temporairement un déplacement de l'appareil de commande (30).

10. Procédé d'exploitation d'un dispositif de préparation de commandes pour médicaments selon l'une des revendications 1 à 9, **caractérisé en ce que** la présence d'un obstacle est signalée à un utilisateur.

11. Dispositif de préparation de commandes (1) pour médicaments, présentant
une pluralité d'emplacements de stockage pour les bouteilles et/ou les emballages de médicaments (6),
un appareil de commande (30) déplaçable horizontalement dans une direction X (axe X) et verticalement dans une direction Z (axe Z) dans un espace de déplacement (20) devant les emplacements de stockage et présentant un préhenseur (31),
au moins un dispositif de stockage (41, 42), avec lequel des bouteilles et/ou des emballages de médicaments (6) peuvent être déplacés dans le dispositif de préparation de commandes (1) et duquel le préhenseur (31) peut prélever des bouteilles et/ou des emballages de médicaments (6),
au moins un dispositif d'identification (51, 52) pour identifier les bouteilles et/ou les emballages de médicaments (6),
un dispositif de déchargement (60) auquel des bouteilles et/ou des emballages de médicaments (6) peuvent être transférés par l'appareil de commande pour être déchargés du dispositif de préparation de commandes (1),
au moins un dispositif de détection optique (34, 70), qui est disposé dans le dispositif de préparation de commandes (1) de telle sorte que celui-ci peut établir une image globale de l'espace de déplacement (20), et
un dispositif de commande (80) couplé à l'appareil de commande (30), au dispositif d'identification (51, 52) et au dispositif de détection optique (34, 70), **caractérisé en ce que** le dispositif de commande est conçu pour exécuter les étapes de procédé selon les revendications 1 à 10.

12. Dispositif de préparation de commandes (1) pour médicaments selon la revendication 11, **caractérisé en ce que** le dispositif de détection optique (34) est un composant du préhenseur (31) d'appareil de commande (30).
